(19) Europäisches Patentamt
European Patent Office
Office européen des brevets

(11) **EP 3 150 211 B1**

(12) **EUROPEAN PATENT SPECIFICATION**

(45) Date of publication and mention
of the grant of the patent:
**15.08.2018 Bulletin 2018/33**

(21) Application number: **15822565.6**

(22) Date of filing: **13.07.2015**

(51) Int Cl.:
*A61K 9/06* (2006.01)         *A61K 9/08* (2006.01)
*A61K 31/737* (2006.01)         *A61P 27/02* (2006.01)

(86) International application number:
**PCT/JP2015/069996**

(87) International publication number:
**WO 2016/009982 (21.01.2016 Gazette 2016/03)**

(54) **THERAPEUTIC AGENT FOR KERATOCONJUNCTIVE DISORDER**

THERAPEUTIKUM FÜR KERATOKONJUNKTIVE ERKRANKUNGEN

AGENT THÉRAPEUTIQUE POUR SOIGNER DES TROUBLES KÉRATOCONJONCTIFS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.07.2014 JP 2014144555**

(43) Date of publication of application:
**05.04.2017 Bulletin 2017/14**

(73) Proprietor: **Santen Pharmaceutical Co., Ltd.
Higashiyodogawa-ku
Osaka-shi
Osaka 533-8651 (JP)**

(72) Inventors:
• **KANEKO, Shinichiro
Kumamoto-shi
Kumamoto 861-8038 (JP)**
• **SASAOKA, Masaaki
Ikoma-shi
Nara 630-0101 (JP)**
• **NAGANO, Takashi
Ikoma-shi
Nara 630-0101 (JP)**
• **SHIRAE, Satoshi
Ikoma-shi
Nara 630-0101 (JP)**

(74) Representative: **Müller-Boré & Partner
Patentanwälte PartG mbB
Friedenheimer Brücke 21
80639 München (DE)**

(56) References cited:
**WO-A1-2009/113435**

• **KOKEN CO., LTD: "Sacran", online , 2005,
XP002769750, Retrieved from the Internet:
URL:http://www.kokenmpc.co.jp/english/prod
ucts/cosmetic_materials/sacran/index.html
[retrieved on 2017-05-02]**
• **DAITO KASEI KOGYO CO., LTD: "Sacran",
online , January 2011 (2011-01), XP002769751,
Retrieved from the Internet:
URL:http://www.daitokasei.com/news/img/SAC
RAN.pdf [retrieved on 2017-05-02]**
• **MICHELE IESTER ET AL: "Improvement of the
ocular surface using hypotonic 0.4% hyaluronic
acid drops in keratoconjunctivitis sicca", EYE,
vol. 14, no. 6, 1 November 2000 (2000-11-01),
pages 892-898, XP055368520, GB ISSN:
0950-222X, DOI: 10.1038/eye.2000.244**
• **TADAHIRO MURAKAMI ET AL.: 'Combined
Effects of Hyaluronan and Artificial Tear Solution
in Rat Dry Eye Model' JOURNAL OF THE EYE vol.
21, no. 1, 2004, pages 87 - 90, XP002501200**
• **'Sacran' (Suizenji Nori Tatotai' FRAGRANCE
JOURNAL vol. 39, no. 1, 15 January 2011, pages
113 - 114, XP008183093**

EP 3 150 211 B1

**Description**

TECHNICAL FIELD

The present invention relates to sacran for use in the treatment or prophylaxis of a keratoconjunctive disorder.

BACKGROUND ART

Sacran is known as a polysaccharide derived from *Aphanothece sacrum.* As applications of sacran, polarization lenses as a polysaccharide-metal ion hybrid liquid crystal gel, photo-trapping agents, photo-scattering agents, flow rate sensors, play toys, actuators, and adhesives have been anticipated, and an anti-oxidation action, an anti-allergic activity and an anti-bacterial property have been reported regarding *Aphanothece sacrum,* thus, applications such as antiviral agents, anti-cancer agents, health promotion agents for use in foods, thickeners and stabilizers for foods, and the like have been anticipated (for example, PTL 1).

A gel-like sustained release carrier and a drug delivery system utilizing the carrier to release the drug has been proposed as an application of sacran (for example, PTL 2).

It has been suggested that sacran has numerous possible applications, and thereamong, it is desirable to search for novel pharmacological applications.

WO 2009/113435 discloses two sugar derivatives obtained from the alga Aphanothece sacrum and the use of these sugar derivatives in the cosmetic field, in foodstuffs, as soil modifiers, as water absorbents for sanitary products, etc. Their pharmaceutical use for gastric-mucosa protection, as anti-cancer agents, anti-coagulant agents, etc. is also disclosed. The list of medical applications includes their use as eyedrops.

Patent Document 1: PCT International Publication No. WO2008/062574
Patent Document 2: Japanese Unexamined Patent Application, Publication No. 2011-68606

DISCLOSURE OF THE INVENTION

Problems to be solved by the Invention

It is the object of the present invention, taking the aforementioned circumstances into consideration, to provide sacran for use in the treatment or prophylaxis of a keratoconjunctive disorder.

Means for Solving the Problems

The present inventors discovered that sacran brings about an excellent improvement effect for corneal disorders in a therapeutic effects test of dry eye and other keratoconjunctive disorders, and completed the present invention.

Specifically, the present invention provides the following:

1. Sacran for use in the treatment or prophylaxis of a keratoconjunctive disorder.

2. Sacran for use according to 1, wherein the keratoconjunctive disorder is at least one selected from the group consisting of keratitis, conjunctivitis and keratoconjunctivitis.

3. Sacran for use according to 1, wherein the keratoconjunctive disorder is at least one selected from the group consisting of dry eye, corneal ulcer, corneal erosion, superficial punctate keratopathy, corneal epithelial defect, conjunctival epithelial defect, conjunctive epithelium disorder, keratoconjunctivitis sicca, superior limbic keratoconjunctivitis and filamentary keratitis.

4. Sacran for use according to any of 1 to 3 which is in the dosage form of an ophthalmic solution or an ophthalmic ointment.

Effects of the Invention

According to the present invention, by comprising sacran, a therapeutic agent and a prophylactic agent for a keratoconjunctive disorder having excellent water storing and water supplying properties when administered to the eye can be provided. Specifically, a therapeutic effects test of corneal disorders was performed, and the sacran in the present invention brings about an excellent therapeutic promoting effect in a corneal disorder model, and thus, effectively operates as a therapeutic agent and a prophylactic agent for keratitis such as corneal ulcer, corneal erosion, superficial punctate keratopathy, and corneal epithelial defect; conjunctivitis such as conjunctival epithelial defect and conjunctive epithelium disorder; and keratoconjunctivitis such as keratoconjunctivitis sicca (dry eye), superior limbic keratoconjunctivitis, and filamentary keratitis.

BRIEF DESCRIPTION OF THE DRAWINGS

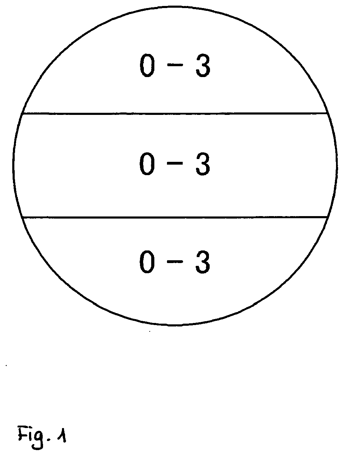

[Fig. 1] is a drawing relating to the determination criteria of the therapeutic effects test of corneal disorders and shows the division of the cornea and the score.

PREFERRED MODE FOR CARRYING OUT THE INVENTION

An embodiment of the present invention will be explained below.

The therapeutic agent and the prophylactic agent for a keratoconjunctive disorder of the present invention is sacran. Sacran is one type of sulfated polysaccharide, has a molecular weight of 2,000,000 or more, and the CAS Registry Number is 1039552-36-7. Sacran, specifically, has a repeat structure of a sugar chain unit where a sugar constituent having a hexose structure and a sugar constituent having a pentose structure are conjugated together in a linear chain or a branched chain through an α-glycoside bond or a β-glycoside bond, the sugar chain unit contains a lactated, sulfated sugar as the sugar constituent, and, in the sugar chain unit, 2.7 or more hydroxyl groups per 100 hydroxyl groups are sulfated or sulfur elements occupy 1.5% by weight or more of all of the elements.

It is known that sacran is obtained by protonating a polysaccharide extracted from *Aphanothece sacrum* which is an endemic species in Japan, and performing partial hydrolysis thereon (for example PTL 1).

Sacran which can be used in the present invention is not limited to sacran derived from *Aphanothece sacrum.*

In the present invention, the keratoconjunctive disorders indicate a state in which the cornea and the conjunctiva have received damage due to various factors. Keratoconjunctive disorders are caused by a reduction in the lacrimal gland due to, for example, dry eye (keratoconjunctivitis sicca), and are caused by injury, autoimmune diseases, drug toxicity and the like.

Keratoconjunctive disorders, from the viewpoint of symptoms, are roughly categorized into keratitis such as corneal ulcer, corneal erosion, superficial punctate keratopathy, and corneal epithelial defect; conjunctivitis such as conjunctival epithelial defect, and conjunctive epithelium disorder. Keratoconjunctive disorders, from the viewpoint of the cause, the site on onset and the symptoms, include keratoconjunctivitis such as keratoconjunctivitis sicca, superior limbic keratoconjunctivitis, and filamentary keratitis. According to the present invention, sacran can be used in all kinds of keratoconjunctive disorders, and thereamong, keratoconjunctivitis sicca is preferable. Keratoconjunctivitis sicca is also known as dry eye.

The sacran for use in the treatment or prophylaxis of a keratoconjunctive disorder of the present invention can be administered parenterally. An ophthalmic solution, an ophthalmic ointment, injections, and the like may be provided as the dosage form, and thereamong, an ophthalmic solution and an ophthalmic ointment are preferred, and the ophthalmic solution is more preferred.

These dosage forms can be prepared using any of the generally used techniques.

For example, the ophthalmic solution can be prepared by using a tonicity agent such as sodium chloride and concentrated glycerin; a buffer agent such as sodium phosphate and sodium acetate; a surfactant such as polyoxyethylene sorbitan monoolate, polyoxyl 40 stearate and polyoxyethylene hydrogenated castor oil; a stabilizing agent such as sodium citrate and sodium edetate; a preservative such as benzalkonium chloride and paraben; and the like as necessary. The pH may be within a range acceptable for ophthalmic drug products, and preferably is in the range of 4 to 8.

The ophthalmic ointment can be prepared with a generally used base such as white petrolatum or liquid paraffin.

The sacran for use in the treatment or prophylaxis of a keratoconjunctive disorder of the present invention is useful as a prophylactic agent, but preferably is a therapeutic agent.

In the sacran for use in the treatment or prophylaxis of a keratoconjunctive disorder of the present invention, specifically, the dosage of sacran can be appropriately selected depending on the symptoms, age, the dosage form, and the like, but in an ophthalmic solution, the sacran concentration may be instilled at 0.00001 to 1%(w/v), preferably 0.0001 to 1%(w/v) one to several times per day, and specifically, one to six times per day, and two or three times per day is preferable.

EXAMPLES

The sacran preparation example, the formulation examples and the results of the pharmacological test are shown below, but these examples are provided for the purpose of better understanding of the present invention, and do not limit the scope of the present invention.

[Preparation example of sacran]

The water-soluble pigment was removed by freezing an appropriate amount of *Aphanothece sacrum* and water, washing after melting, and next, the liposoluble pigment was removed while stirring overnight in ethanol. After separating the ethanol from *Aphanothece sacrum* from which these pigments were removed, the solution was heated in a 0.4-0.6N aqueous solution of sodium hydroxide at 40°C, and dissolved while stirring over approximately five hours. An aqueous solution of the sugar derivative extracted by the operation was neutralized with hydrochloric acid and desalinated by soaking in 60 to 80% isopropyl alcohol. After desalinization, the concentrate was poured into 100% isopropanol under agitation and a gel-like sacran precipitated, and the gel-like sacran was dried by hot air at 85°C or higher for six hours to obtain sacran (the present compound) derived from *Aphanothece sacrum* which is a fibrous sugar derivative.

[Formulation examples]

Typical formulation examples which used sacran (the present compound) are shown below.

[Formulation example 1] Ophthalmic solution

| In 100 ml | |
|---|---|
| Present compound | 200 mg |
| Sodium chloride | 900 mg |
| Sterile purified water | Appropriate amount |

[Formulation example 2] Ophthalmic solution

| In 100 ml | |
|---|---|
| Present compound | 200 mg |
| Sodium chloride | 800 mg |
| Disodium hydrogenphosphate | 100 mg |
| Sodium dihydrogenphosphate | Appropriate amount |
| Sterile purified water | Appropriate amount |

[Formulation example 3] Ophthalmic ointment

| In 100 g | |
|---|---|
| Present compound | 0.2 g |
| Liquid paraffin | 10.0 g |
| White petrolatum | Appropriate amount |

[Pharmacological test]

1. Therapeutic effects test of corneal disorders

Using a male SD rat, a corneal disorder model was produced according to the method of Fujihara, et al. (Invest. Ophthalmol. Vis. Sci 42(1): 96-100(2001)). After producing the corneal disorder model, the extent of the corneal disorder was scored according to the method of Murakami, et al. (Atarashii Ganka (New Ophthalmology) 21(1):87-90. (2004)).

(Test method)

A male SD rat was systematically anesthetized by an interperitoneal administration of 2.5 mL/kg Somnopentyl injection diluted five-fold with a physiological saline solution, and maintained in the anesthetized state by inhalation of (1.5%) Escain. Next, an incision was made on both sides of the buccal skin, the exorbital lacrimal gland was removed, and the skin was sutured by a Michel suture clip. Then, corneal damage was induced over eight weeks. Further, normal animals in which the removal of the lacrimal gland was not performed were prepared.

Next, 5 μL of the phosphate buffer solution (PBS) was instilled six times per day for four weeks in both eyes (normal eye) in the normal animals, and 5 μL of PBS (control), or a 0.006% or a 0.2% PBS solution of the present compound were instilled six times per day for four weeks in both eyes in the corneal disorder model. The cornea was stained with fluorescein prior to and four weeks after the initiation of instillation. The upper, middle and lower parts of the cornea were assessed for the extent of staining with fluorescein according to the following criteria, and the extent of the corneal damage was calculated from the mean value of the total scores of the aforementioned respective parts.

The normal eyes were tested in the same manner as stated above and the mean value of the total scores of the aforementioned respective parts was sought.

(Determination criteria)

1) The cornea was divided into upper, middle and lower parts as shown in Fig. 1, and a score from 0 to 3 was conferred on the basis of the criteria of Table 1. 0.5 was provided as an intermediate value.

2) The scores of the three regions were totaled, and a defect score (0-9) was calculated for each observed eye.

[Table 1]

| Score | Determination criteria |
|-------|------------------------|
| 0 | No punctate staining |
| 1 | Scattered staining (Fluorescein staining of punctates separated) |
| 2 | Intermediate staining (Intermediate between 1 and 3) |
| 3 | Heavy staining (Fluorescein staining of punctates nearly abut) |

(Results)

The improvement ratio due to the instillation of the present compound calculated on the basis of the mean value of the total scores of the aforementioned respective parts in the control group as a standard value (improvement ratio: 0%) according to the following calculation formula is shown in Table 2. Note that, the mean value of the scores is the average of 8 eyes (4 animals).

$$\text{Improvement ratio (\%)} = \{(\text{control}) - (\text{present compound})\} / \text{damage degree} \times 100$$

$$\text{Damage degree} = \{(\text{control}) - (\text{normal eye})\}$$

[Table 2]

| Group | Four weeks after start of ocular instillation | |
|-------|-----------------------------------------------|--|
| | Mean value of total scores | Improvement ratio (%) |
| Normal eye | 2.7 | |

(continued)

| Group | Four weeks after start of ocular instillation | |
| --- | --- | --- |
| | Mean value of total scores | Improvement ratio (%) |
| Control | 5.6 | 0 |
| 0.006% of present compound | 4.4 | 41 |
| 0.2% of present compound | 2.7 | 100 |

(Discussion)

As is apparent from the results of the pharmacological test in which the aforementioned rat was used, the present compound markedly improves the corneal disorders.

2. Therapeutic effects test of corneal disorders (Examination of the instillation frequency)

The aforementioned test results were examined by the same test method regarding the influence of the instillation frequency of a 0.2% PBS solution of the present compound on the improvement result on corneal disorders.

(Test method)

Corneal damage was induced over eight weeks by performing the same operation as in the aforementioned test to the male SD rats. Further, normal animals in which the removal of the lacrimal glands was not performed were prepared.
Next, 5 $\mu$L of PBS (normal eye) was instilled six times per day for four weeks in both eyes in the normal animal, 5 $\mu$L of PBS (control) was instilled six times per day for four weeks in both eyes and 5 $\mu$L of 0.2% PBS solution of the present compound was instilled two or six times per day for four weeks in both eyes in the corneal disorder model.
The cornea was stained with fluorescein prior to and one, two, and four weeks after the initiation of instillation, and the extent of the corneal disorder was calculated by the same methods as the aforementioned test.
The normal eye was tested in the same manner as stated above and the mean value of the total scores of the aforementioned respective parts was sought.

(Results)

The improvement ratio due to the instillation of the present compound calculated in the same manner as the improvement effect in the aforementioned test is shown in Table 3. Note that, the mean value of the scores is the average of 8 eyes (4 animals).

[Table 3]

| Group | One week after start of ocular instillation | | Two weeks after start of ocular instillation | | Four weeks after start of ocular instillation | |
| --- | --- | --- | --- | --- | --- | --- |
| | Mean value of total scores | Improvement ratio (%) | Mean value of total scores | Improvement ratio (%) | Mean value of total scores | Improvement ratio (%) |
| Normal eye | 2.4 | | 3.5 | | 3.6 | |
| Control | 5.8 | 0 | 5.2 | 0 | 6.0 | 0 |
| Ocular instillation two times per day | 4.0 | 53 | 3.5 | 100 | 3.8 | 92 |
| Ocular instillation six times per day | 3.4 | 71 | 3.3 | 112 | 3.0 | 125 |

(Discussion)

As is apparent from the results of the pharmacological test in which the aforementioned rat was used, the present compound markedly improves the corneal disorders, and the improvement effect was observed even with ocular instillation two times per day, but specifically, the improvement effect could be observed from the early stage when ocular instillation was performed six times per day.

3. Evaluation testing of the conjunctive epithelium disorder improvement action

The aforementioned test results were examined regarding the improvement effect on conjunctive epithelium disorder (reduction of conjunctival goblet cells) of a 0.2% PBS solution of the present compound.

(Test method)

Corneal damage was induced over eight weeks by performing the same operation as in the aforementioned test to the male SD rats. Further, normal animals in which the removal of the lacrimal glands was not performed were prepared.
Next, 5 μL of PBS (normal eye) was instilled six times per day for four weeks in both eyes in the normal animal, 5 μL of PBS (control) was instilled six times per day for four weeks in both eyes and 5 μL of 0.2% PBS solution of the present compound was instilled two or six times per day for four weeks in both eyes in the conjunctive epithelium disorder model.
The eyelids were excised four weeks after the start of ocular instillation and a histopathologic examination was performed. After the excised eyelids were immersed and fixed in 10% neutral buffered formalin, and the paraffin substituted, the ear sides were made as an embedded surface to prepare a block, and thin cutting was performed to produce a PAS reaction sample.
Using a 40X objective lens, the number of all of the conjunctival goblet cells observed in the samples were counted for the respective upper and lower eyelids.

(Results)

The effect for increasing the conjunctival goblet cells by continuous instillation of the present compound for four weeks is shown in Table 4. Note that, the mean value of the number of goblet cells is the average of 7-8 eyes (4 animals).

[Table 4]

| Group | Number | Number of goblet cells (Mean value) |
|---|---|---|
| Normal eye | 8 eyes | 583 |
| Control | 8 eyes | 446 |
| 0.2% of present compound Ocular instillation two times per day | 7 eyes | 551 |
| 0.2% of present compound Ocular instillation six times per day | 7 eyes | 514 |

(Discussion)

From the aforementioned histopathological test results, the present compound has an effect for improving conjunctive epithelium disorders caused by the removal of the lacrimal glands, and this improvement effect was observed not only with ocular instillation six times per day, but also with ocular instillation two times per day.

**Claims**

1. Sacran for use in the treatment or prophylaxis of a keratoconjunctive disorder.

2. Sacran for use according to claim 1, wherein the keratoconjunctive disorder is at least one selected from the group consisting of keratitis, conjunctivitis and keratoconjunctivitis.

3. Sacran for use according to claim 1, wherein the keratoconjunctive disorder is at least one selected from the group consisting of dry eye, corneal ulcer, corneal erosion, superficial punctate keratopathy, corneal epithelial defect, conjunctival epithelial defect, conjunctive epithelium disorder, keratoconjunctivitis sicca, superior limbic keratoconjunctivitis and filamentary keratitis.

4. Sacran for use according to any one of claims 1 to 3 which is in the dosage form of an ophthalmic solution or an ophthalmic ointment.

**Patentansprüche**

1. Sacran zur Verwendung in der Behandlung oder Prophylaxe einer keratokonjunktiven Störung.

2. Sacran zur Verwendung nach Anspruch 1, wobei die keratokonjunktive Störung mindestens eine ist, ausgewählt aus der Gruppe, bestehend aus Keratitis, Konjunktivitis und Keratokonjunktivitis.

3. Sacran zur Verwendung nach Anspruch 1, wobei die keratokonjunktive Störung mindestens eine ist, ausgewählt aus der Gruppe, bestehend aus trockenem Auge, Hornhautgeschwür, Hornhauterosion, oberflächlicher punktförmiger Keratopathie, kornealem Epitheldefekt, Bindehautepitheldefekt, Konjunktivepithelerkrankung, Keratokonjunktivitis sicca, übergeordneter limbischer Keratokonjunktivitis und fadenförmiger Keratitis.

4. Sacran zur Verwendung nach einem der Ansprüche 1 bis 3, welches in der Dosierungsform einer ophthalmischen Lösung oder einer ophthalmischen Salbe vorliegt.

**Revendications**

1. Sacran destiné à être utilisé dans le traitement ou la prophylaxie d'un trouble kératoconjonctif.

2. Sacran destiné à être utilisé selon la revendication 1, dans lequel le trouble kératoconjonctif est au moins un sélectionné parmi le groupe constitué de la kératite, conjonctivite et kératoconjonctivite.

3. Sacran destiné à être utilisé selon la revendication 1, dans lequel le trouble kératoconjonctif est au moins un sélectionné parmi le groupe constitué de l'oeil sec, d'un ulcère cornéen, d'une érosion cornéenne, d'une kératopathie ponctuée superficielle, d'un défaut épithélial cornéen, d'un défaut épithélial conjonctival, d'un trouble épithélial conjonctif, d'une kératoconjonctivite sèche, d'une kératoconjonctivite limbique supérieure et d'une kératite filamenteuse.

4. Sacran destiné à être utilisé selon l'une quelconque des revendications 1 à 3, qui est sous la forme de dosage d'une solution ophtalmique ou d'une pommade ophtalmique.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009113435 A
- WO 2008062574 A
- JP 2011068606 A

**Non-patent literature cited in the description**

- *CHEMICAL ABSTRACTS,* 1039552-36-7
- **FUJIHARA et al.** *Invest. Ophthalmol. Vis. Sci,* 2001, vol. 42 (1), 96-100
- **MURAKAMI ; ATARASHII GANKA et al.** *New Ophthalmology,* 2004, vol. 21 (1), 87-90